# EUROPEAN PATENT SPECIFICATION

(11) **EP 3 217 915 B1**
(45) Date of publication and mention of the grant of the patent: **23.08.2023**
(21) Application number: 15818032.3
(22) Date of filing: 11.11.2015
(51) Int. Cl.: A61B 50/30, A61B 90/00

(54) **MACHINE FOR COUNTING MEDICAL ITEMS AND CORRESPONDING METHOD**
MASCHINE ZUM ZÄHLEN MEDIZINISCHER ARTIKEL UND ENTSPRECHENDES VERFAHREN
MACHINE PERMETTANT DE COMPTER DES ARTICLES MÉDICAUX ET PROCÉDÉ CORRESPONDANT

(30) Priority: 11.11.2014 IT UD20140176
(43) Date of publication of application: 20.09.2017
(73) Proprietor: Infinisurge S.r.l., 33100 Udine (IT)
(72) Inventor: DREOSSO, Claudio, 33100 Udine (IT); DREOSSO, Alessandro, 33035 Martignacco (IT)
(74) Representative: Petraz, Davide Luigi
(86) International application number: PCT/IB2015/058710
(87) International publication number: WO 2016/075634

(56) References cited:
- EP-A2- 2 218 421
- WO-A1-89/09563
- FR-A1- 2 598 086
- FR-A1- 2 714 896
- FR-A1- 2 749 569
- US-A- 4 911 294
- US-A- 5 227 765
- US-A1- 2005 075 564
- US-A1- 2008 024 276
- US-A1- 2014 262 553

## Description

### FIELD OF THE INVENTION

The present invention concerns a machine for counting medical items, such as surgical sponges used in hospitals during surgery.

The present invention also concerns a method for counting medical items.

### BACKGROUND OF THE INVENTION

It is known to use medical articles, for example surgical sponges, during surgical operations in operating theaters in hospitals.

Before a surgical operation, the sterile surgical sponges introduced into the operating theater are counted, to check the number.

At the end of the surgical operation, the surgical sponges remaining and the surgical sponges used are counted again, to exclude the possibility that one or more surgical sponges have been accidentally left inside a patient.

The surgical sponges can be counted either manually or electronically.

If they are counted manually, i.e. by an operator, the number can be falsified by the operator's urgency and/or haste, before and/or during the surgical operation, and this represents a percentage of error unacceptable in this field.

For electronic counts, surgical sponges with identification codes, or microtags, are known, the data of which is acquired by a surgical sponge counting machine.

Known surgical sponge counting machines generally comprise a slider on which a control and management unit is installed, configured to control and manage the stream and consequently to count the surgical sponges.

These known machines also have a container installed on the slider into which the used surgical sponges are introduced. One example of this solution is described in US-A-2014/0262553.

The container is provided with an aperture facing upward, which can entail an involuntary introduction of surgical sponges or other objects into the container.

It is also known that the control and management unit is connected to at least one detection device, such as a scanner enabled to detect and read the identification codes of the surgical sponges.

If there is only one detection device, it is associated with the container for collecting the used surgical sponges, and only counts the surgical sponges that are introduced into the container on each occasion. Before starting a surgical operation, the surgical sponges are counted manually by an operator who introduces the number of surgical sponges he/she has counted into the control and management unit by means of suitable interfaces. This number is decreased every time a surgical sponge is introduced into the container. At the end of the surgical operation the control and management unit will supply to the operator, for example on the graphical interface, the number of surgical sponges that have been introduced into the container.

The operator is therefore required to count the remaining surgical sponges again and to check that these, added to the number of surgical sponges introduced into the container, correspond to the number of surgical sponges originally supplied in the operating theater.

One disadvantage of such control and management units is that they are not able to supply instantaneously the operators with a report on the stream of surgical sponges in an operating theater, i.e. on the number of surgical sponges supplied, the number of surgical sponges used during the surgical operation, and the number of surgical sponges remaining and unused at the end of the surgical operation.

This report is of fundamental importance, especially in its written form, if disputes arise, for example for problems to or deaths of patients, and can constitute certain proof in a lawsuit.

Even if written reports can be prepared manually by the operator, they are not reliable and can be subject to tampering.

One solution is known, described in WO-A-89/09563, which discloses the features of the preamble of claim 1, providing an optical detection of the surgical sponges that are introduced on each occasion into the container. This solution is affected by errors, however, if an operator introduces several surgical sponges simultaneously, since in this case the optical detector can detect only one surgical sponge, even if in fact several surgical sponges have been introduced.

Solutions are also known in which there is a first detection device to make an initial count of the sterile surgical sponges introduced into the operating theater, and a second detection device to count the used surgical sponges introduced into the container during the surgical operation.

The first detection device comprises a portable reader suitable to read codes, RFID or other tags.

The second detection device is usually configured as a ring and is installed near the aperture of the container so as to surround it. In this way the second detection device is able to count on each occasion the surgical sponges that are introduced into the container, detecting the identification codes associated with each surgical sponge.

Ring-type detection devices do not ensure that the surgical sponges are correctly counted, for example because of a too rapid introduction and/or overlapping surgical sponges in the container.

It is also known that known surgical sponge counting machines provide to install individual components on the slider as separate entities, which can have protruding portions which the operators can get caught on and can knock against, and/or shaped portions that are difficult to clean or on which the operators can get caught.

The data collected by the control and management unit, moreover, are only used during the surgical operation and they are eliminated at the end of the operation. The control and management unit is therefore a unit in itself which is not able to interact with the outside.

Known counting machines are generally light and have limited stability if an operator knocks against them.

One purpose of the present invention is to obtain a machine for counting surgical sponges that allows to provide the operators with evidence of the stream of medical articles inside the workplace, for example the hospital.

Another purpose of the present invention is to obtain an extremely reliable machine that limits, and even eliminates, disadvantages connected to an erroneous surgical sponge count.

Another purpose of the present invention is to obtain a machine that is simple and intuitive to use by operators.

Another purpose of the present invention is to obtain a machine for counting surgical sponges that is extremely compact and stable.

Another purpose of the present invention is to obtain a machine that allows to exchange information with external units, to control and manage the stream of medical articles in an operational environment.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, a machine for counting medical articles is according to annexed claim 1.

The present solution allows, for example at the end of a surgical operation, to obtain a written report, which cannot easily be tampered with by the operators, on the stream of medical articles such as surgical sponges, which have been used during the surgical operation.

The written report obtained can be attached, for example, to the patient's medical record and, if possible disputes arise, can constitute certain proof of the surgical sponges used during the surgical operation, excluding the possibility that any surgical sponges can have remained inside a patient.

This solution also allows to obtain a machine for counting medical articles in which the count is carried out with extreme accuracy and cannot voluntarily be falsified by the operators.

The box-like shape of the support structure ensures that the only access to the container is the one defined by the introduction aperture, and using an RFID reader ensures that the simultaneous introduction of several medical articles is in any case detected, even if these are grouped together.

The box-like shape of the support structure also ensures that the content of the medical articles in the container is not voluntarily tampered with by the operators, thus guaranteeing that the data printed by the printing device are correct; in fact, the printed data constitutes for example a document with a legal value for a surgical operation.

As is evident, this purpose can be obtained by the synergic combination of the support structure with box-like shape, the printing device and the association of the RFID reader with the introduction aperture for the accurate detection of the passage of each medical article.

The present invention also concerns a method for counting medical articles according to annexed claim 8.

### BRIEF DESCRIPTION OF THE DRAWINGS

These and other characteristics of the present invention will become apparent from the following description of some forms of embodiment, given as a non-restrictive example with reference to the attached drawings wherein:
- fig. 1 is a perspective view of a machine for counting medical articles, in accordance with a possible form of embodiment of the present invention;
- fig. 2 is a schematic view in lateral section of the electronic machine in fig. 1, in accordance with the present invention.

To facilitate comprehension, the same reference numbers have been used, where possible, to identify identical common elements in the drawings.

### DETAILED DESCRIPTION OF SOME FORMS OF EMBODIMENT

We shall now refer in detail to the various forms of embodiment of the present invention, of which one or more examples are shown in the attached drawing. Each example is supplied by way of illustration of the invention and shall not be understood as a limitation thereof.

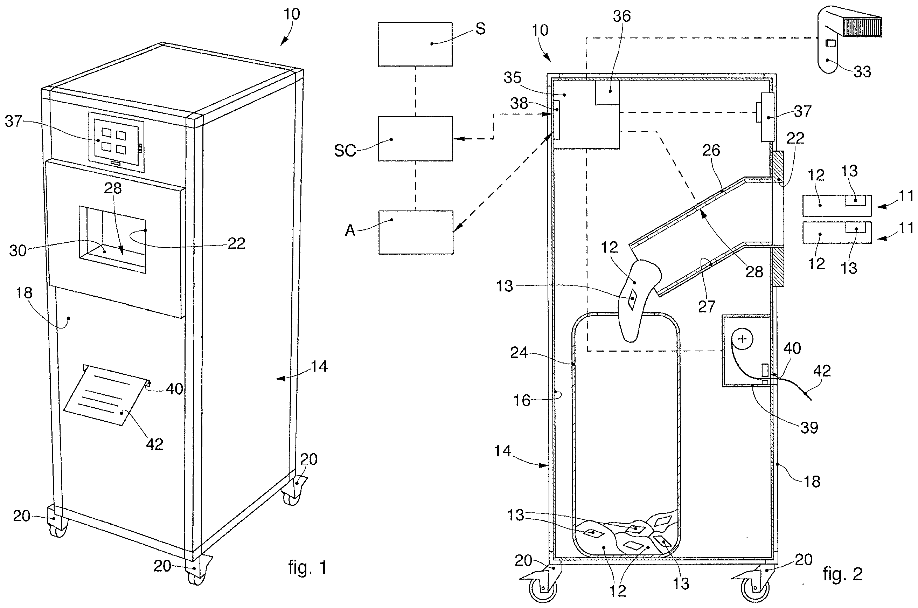

According to the present invention, figs. 1-2 are used to describe forms of embodiment of a machine 10 for counting medical articles 12.

According to a possible solution, the machine 10 allows to count medical articles such as surgical sponges 12, which are usually used in the hospital field.

The machine 10 comprises a support structure 14 configured to support functional components of the machine as will be described hereafter.

As shown in figs. 1 and 2, the support structure 14 has a substantially box-like shape, hollow inside, to define a containing compartment 16 into which, as will be described hereafter, at least some of the functional components of the machine 10 are disposed.

According to the present invention, the support structure 14 can be about 150 cm high, 50 cm wide and 50 cm long.

According to one form of embodiment of the present invention, the support structure 14 can have a substantially parallelepiped shape defined by a plurality of flat walls, reciprocally connected with each other.

In this way the support structure 14 has a substantially linear configuration, without any protruding portions which the operators can get caught on.

Furthermore, this configuration makes the operations of cleaning the machine 10 extremely simple, thus ensuring that the machine is sterile.

According to a possible form of embodiment, the support structure 14 can be provided with movement means 20, installed on the lower wall of the support structure 14, and which allow to move it and transfer it to the zones where it is used.

The movement means 20 can be wheels, for example.

The support structure 14 is provided with at least an introduction aperture 22 which allows accessibility from outside to the containing compartment 16.

According to a possible solution, the introduction aperture 22 defines the only aperture for access to the containing compartment 16 and the container 24, thus preventing any tampering or accidental introduction of surgical sponges 12 into the latter.

According to a possible solution, the support structure 14 comprises a front wall 18, and the introduction aperture 22 is made in the front wall 18 to allow the introduction of the surgical sponges 12.

The front wall 18 is located substantially vertical or sub-vertical during use, and the introduction aperture 22 is made in the thickness thereof.

In this way any involuntary and/or accidental introduction of the surgical sponges 12 is prevented, for example due to them falling, and therefore an incorrect count is prevented. In fact, when the surgical sponge 12 has to be introduced into the containing compartment 16, the operator has to throw and/or introduce the surgical sponge 12 deliberately through the introduction aperture 22.

One or more containers 24 for the surgical sponges 12 introduced can be disposed in the containing compartment 16, which is accessible from the introduction aperture 22.

The container 24 can be the rigid type as shown in fig. 2, or the flexible type, for example a bag installed on a suitable support element.

A conveyor pipe 26 is associated with the support structure 14, attached in correspondence with the introduction aperture 22 and configured to convey the surgical sponges 12 that are introduced through the latter to the container 24.

The conveyor pipe 26 allows to ensure that an operator cannot voluntarily access directly into the container 24, through the introduction aperture 22, in order to tamper with the content inserted therein.

The conveyor pipe 26 has a tubular shape defined by a through cavity 27 with a shape of the cross section at least the same as that of the introduction aperture 22.

The conveyor pipe 26 is provided with a discharge end through which the surgical sponges 12 are discharged to the container 24.

According to possible solutions, if the container 24 is the flexible type, like a bag, it can be attached directly to the discharge end of the conveyor pipe 26, for example by attachment means.

The machine 10 according to the present invention comprises at least a first detection device 28 configured to detect at least the number of surgical sponges 12 which on each occasion are introduced into the container 24 through the introduction aperture 22.

The surgical sponges 12 are provided with an RFID identification element 13, selectively detectable by the first detection device 28.

The identification element 13 can be univocal for each surgical sponge 12, so that a predefined code can be associated with each of them, and it is possible to prevent multiple counts of the surgical sponges 12.

As shown in figs. 1 and 2, the first detection device 28 comprises an RFID reader, suitable to detect the presence of the identification element 13 of the surgical sponge 12.

The first detection device 28 is associated with the through cavity 27 of the conveyor pipe 26 and extends for the entire surface development thereof.

This solution is extremely effective with regard to detecting the surgical sponges 12 that are introduced on each occasion into the container 24. In fact, the whole time the surgical sponge 12 is passing through the conveyor pipe 26, the first detection device 28 is able to detect the identification element 13 associated with the surgical sponge 12. This prevents the surgical sponge from being detected if it is introduced through the introduction aperture 22 too quickly.

The first detection device 28 can allow to count the surgical sponges 12 that are thrown into the container 24 on each occasion.

However, it is not excluded that, in possible solutions that will be described hereafter in the description, the first detection device 28 can also be used to make an initial count of the surgical sponges 12, for example as soon as they are introduced into the operating theater.

In this case, the surgical sponges 12 are introduced and removed manually by the operator through the introduction aperture 22 so that the first detection device 28 detects their presence and identifies at least the number of surgical sponges 12 that have been introduced.

The univocal nature of the code associated with each identification element 13 prevents double loading of the surgical sponges 12.

The machine 10 also comprises a control and management unit 35 connected at least to the first detection device 28 and configured to control and manage at least the stream of surgical sponges 12 introduced into the container 24.

In particular, the control and management unit 35 is configured at least to detect the data that are supplied on each occasion by the first detection device 28.

For example, it can be provided that the control and management unit 35 at least temporarily memorizes and controls the identification codes transmitted by the first detection device 28, for example to verify a double count of the surgical sponges 12 introduced.

According to other forms of embodiment, the control and management unit 35 can implement at least one management program of the first detection device 28 and the information relating to the identification codes contained in the identification elements 13.

In particular, the control and management unit 35 can implement a first program, or surgical sponge 12 loading program, configured to allow an operator to detect the number of surgical sponges 12 that are available in an operating theater, for example before the surgical operation starts. Once the loading program has been started, the operator makes the first detection device 28 read the identification codes of the surgical sponges, temporarily introducing and removing the surgical sponges from the introduction aperture 22.

Once the loading program has finished, a second program, or surgical sponge discharge program, can be started in the control and management unit 35: this is suitable to count the number of surgical sponges 12 inserted into the container 24 on each occasion.

According to possible solutions of the invention, the machine 10 can be provided with a user interface 37, for example a touchscreen, with which an operator can interface at least to know the number of surgical sponges 12 that have been inserted into the container 24.

The user interface 37 can be installed in the support structure 14, for example near its front wall 18.

The user interface 37 is connected to the control and management unit 35 and can also allow the operator to start or terminate at least the loading and unloading programs described above.

The control and management unit 35 can be provided with an internal memory 36 to memorize an archive of the stream of surgical sponges 12 during surgical operations. In particular, the archive of the streams of surgical sponges 12 can include data relating to the initial and final counts of the surgical sponges 12, the type of surgical sponges 12, the patient, the staff present in the operating theater or space-time data needed to trace the different surgical operations.

The control and management unit 35 also comprises a printing device 39 configured to print on a print support 42, for example paper, data relating at least to the stream of surgical sponges 12 introduced into the container 24, and also possible other data as described above.

The printing device 39 is positioned in the containing compartment 16 of the support structure 14.

The support structure 14 is provided with a slit 40 for the emission of the printed print support 42. The slit 40 can be made directly in the front wall 18 of the support structure 14.

The possibility of printing a written report of the streams of surgical sponges 12 in the operating place of the machine 10 allows to supply written proof, which cannot be tampered with by the operators, possibly used during lawsuits, as a confirmation of the correct procedures followed during the surgical operation, at least with regard to the streams of surgical sponges 12.

According to possible solutions, the control and management unit 35 can be provided with at least a communication interface 38, configured to transmit the data collected in the control and management unit 35 to external processing units.

Merely by way of example, the communication interface 38 can comprise connectors or bus ports.

The communication interface 38 can be connected by connectors, for example a LAN connection or other connection modes, or wireless mode, to units outside the control and management unit 35.

According to a possible solution, the control and management unit 35 can be connected by the communication interface 38 to a central unit SC, for example the hospital's, to supply indications relating to the stream of surgical sponges 12 processed by the machine 10.

The data relating to streams of surgical sponges used by more than one machine 10 according to the present invention can also converge into the central unit SC, thus supplying the capacity to manage the reserves of surgical sponges 12 in stock. To this purpose the central unit SC can be connected to at least a reserve management unit S to provide indications also relating to a possible supply of surgical sponges both to the store itself and also to the individual machines 10 according to the present invention.

The central unit SC can in turn be connected to assistance centers A, which through connection can perform, directly from their stations, possible maintenance interventions on the individual machines 10 according to the present invention.

In this way the assistance center, for some simple interventions, for example software updates, program malfunctions and/or backup, can act directly from remote without the direct intervention of operators.

According to a possible form of embodiment of the present invention, the control and management unit 35 can be connected to a second detection device 33 outside the support structure 14 and configured to perform an initial count of the sterile surgical sponges 12.

The second detection device 33, for example an RFID detector or a detector of a similar type to that of the first detection device 28, can be connected to the control and management unit 35 by a wireless connection.

The second detection device 33 can be the portable type, to allow it to be maneuvered by the user to perform an initial count of the surgical sponges 12.

In this case, the data detected by the second detection device 33 are managed by the control and management unit 35, for example during the loading program as described above.

According to possible solutions, the control and management unit 35 is connected to alarm devices suitable to supply acoustic and/or visual alarms if an incorrect loading of the surgical sponges 12 is performed, for example in the event of double counting of the same surgical sponge 12, and hence a double detection of the same identification element 13, and/or because unidentifiable objects have been inserted into the container 24.

At least the first detection device 28, the control and management unit 35, the container 24 and the printing device 39 are contained inside the containing compartment 16. This allows to obtain an extremely compact machine 10 in which the individual parts are not exposed to a potentially contaminated environment. Furthermore, it allows to simplify the conformation of the support structure 14, making the cleaning operations much simpler.

The machine 10 for counting surgical sponges 12 functions as follows.

Before the surgical operation, the operating theater receives a predefined number of sterile surgical sponges 12 contained, for example, inside a blister 11. Each surgical sponge 12 has a different identification element 13.

The operator selects the initial surgical sponge 12 count function by means of the user interface 37. For the initial count, each surgical sponge 12 is passed only in proximity to the introduction aperture 22 and consequently the first detection device 28, so that the corresponding identification element 13 can be read and detected. Every time the first detection device 28 detects a surgical sponge 12, the control and management unit 35 increases the number of surgical sponges 12 loaded.

The initial counting operation can possibly be carried out by the second detection device 33 if it is provided.

In one form of embodiment of the present invention, the machine 10 can provide acoustic signals every time a surgical sponge 12 is read and detected by the first detection device 28.

When the number of sterile surgical sponges 12 loaded has been verified, the operator selects the used surgical sponge 12 count function, using the user interface 37.

Once the surgical operation has begun, every time a used surgical sponge 12 is thrown into the introduction aperture 22, the count of the used surgical sponges 12 is increased.

Once thrown away, the surgical sponges 12 are housed in the container 24 and isolated from the outside.

By means of the machine 10, the one or more operators present in the operating theater can know, and hence verify in real time, the number of surgical sponges 12 used and not used.

Once the surgical operation has been concluded, an operator can print a written report of the surgical operation, by means of the printing device 39, and this can be attached for example to the patient's medical record.

Before using the machine 10 again for a new surgical operation, it can be provided to replace the container by a new container 24. To this purpose the support structure 14 can be provided with an aperture to access the containing compartment 16 where the container 24 is disposed.

In a possible form of embodiment of the present invention, the machine 10 can be provided with a covering layer attached in a selectively removable manner to the conveyor pipe 26 and which covers the through cavity 27 to prevent the conveyor pipe 26 from getting dirty. It can be removed and replaced by a new covering layer before each surgical operation.

## Claims

1. Machine for counting medical items such as surgical sponges used during surgery (12), comprising at least a support structure (14) configured to support at least a container (24) into which, during use, said medical items (12) are introduced, a first detection device (28) configured to detect at least a stream of said medical items (12) to said container (24) and a control and management unit (35) connected at least to said first detection device (28) to acquire data at least relating to said stream of medical items (12) to said container (24), and comprising a printing device (39) configured to print on a print support (42) at least some of said data relating at least to said stream of medical items (12) introduced into said container (24), said support structure (14) having a substantially box-like shape, hollow internally, to define a containing compartment (16) in which at least said container (24) is disposed, said support structure (14) being provided with an introduction aperture (22) configured to allow accessibility to said container (24) and through which, during use, said medical items (12) are introduced, **characterized in that** said first detection device (28), said control and management unit (35) and said printing device (39) are disposed in said containing compartment (16), said support structure (14) being provided with a slit (40) for the emission of said print support (42) when it has been printed, **in that** said introduction aperture (22) defines the only aperture for access to the containing compartment (16) and the container (24), **in that** a conveyor pipe (26) is associated with said support structure (14), attached in correspondence with the introduction aperture (22) and configured to convey said medical items (12) to said container (24), **in that** said conveyor pipe (26) has a tubular shape defined by a through cavity (27) **and in that** said first detection device (28) comprises an RFID reader configured to detect the passage of each RFID identifying element (13) associated with each of said medical items (12), said first detection device (28) being associated with said through cavity (27), extending for the whole of its surface development.

2. Machine as in claim 1, **characterized in that** said control and management unit (35) is connected to a second detection device (33) outside said support structure (14) and comprising an RFID detector to count said medical items (12).

3. Machine as in claim 2, **characterized in that** said second detection device (33) is the portable type.

4. Machine as in any claim hereinbefore, **characterized in that** said support structure (14) comprises a front wall (18) located in a vertical or substantially-vertical position, and said introduction aperture (22) is made in said front wall (18).

5. Machine as in any claim hereinbefore, **characterized in that** it comprises a user interface (37) installed in the support structure (14), connected to said control and management unit (35) and configured to display said data on the stream of medical items (12).

6. Machine as in any claim hereinbefore, **characterized in that** said control and management unit (35) is provided with an internal memory (36) to memorize an archive of the streams of said medical items (12).

7. Machine as in any claim hereinbefore, **characterized in that** said control and management unit (35) is provided with at least a communication interface (38), configured to transmit the data collected in the control and management unit (35) to external processing units.

8. Method for counting medical items (12), which provides to introduce medical items (12) into a container (24) associated with a support structure (14), to detect, by means of a first detection device (28), at least a stream of said medical items (12) to said container (24), to acquire data relating to said stream of medical items (12) to said container (24) by means of a control and management unit (35) connected to said first detection device (28), and to print on a print support (42), by means of a printing device (39), at least some of said data relating at least to said stream of medical items (12) introduced into said container (24), **characterized in that** said detection of said stream of said medical items (12) occurs by means of an RFID reader that detects the passage of each RFID identification element (13) associated with each of said medical items (12) through an introduction aperture (22) provided in said support structure (14) with a substantially box-like shape, internally hollow, in order to define a containing compartment (16) in which at least said container (24) is disposed, **in that** said first detection device (28), said control and management unit (35) and said printing device (39) are disposed in said containing compartment (16), said print support (42), when it has been printed, being emitted through a slit provided in said support structure (14), **and in that** said detection of the stream of medical items (12) occurs along the entire extension of a conveyor pipe (26) associated with the support structure (14) in correspondence with the introduction aperture (22), and which conveys said medical items (12) to said container (24).

## Patentansprüche

1. Maschine zum Zählen medizinischer Artikel (12), wie chirurgischer Schwämme, die während eines chirurgischen Eingriffs verwendet werden, umfassend mindestens eine Trägerstruktur (14), die dafür eingerichtet ist, um mindestens einen Behälter (24) zu tragen, in den, im Gebrauch, die genannten medizinischen Artikel (12) eingeführt werden, eine erste Detektionsvorrichtung (28), die dafür eingerichtet ist, um mindestens einen Zustrom der genannten medizinischen Artikel (12) zu dem genannten Behälter (24) zu detektieren, und eine Steuer- und Verwaltungseinheit (35), die mindestens mit der genannten einen ersten Detektionsvorrichtung (28) verbunden ist, um Daten mindestens in Bezug auf den genannten Zustrom medizinischer Artikel (12) zu dem genannten Behälter (24) zu erfassen, und umfassend eine Druckvorrichtung (39), die dafür eingerichtet ist, um auf einen Druckträger (42) mindestens einige der genannten Daten in Bezug auf mindestens den genannten Zustrom medizinischer Artikel (12) zu drucken, die in den genannten Behälter (24) eingeführt werden, wobei die genannte Trägerstruktur (14) eine im Wesentlichen kastenartige Form aufweist, innen hohl, um ein Halteabteil (16) zu definieren, in dem mindestens der genannte Behälter (24) angeordnet ist, wobei die genannte Trägerstruktur (14) mit einer Einführöffnung (22) versehen ist, die dafür eingerichtet ist, um eine Zugänglichkeit zu dem genannten Behälter (24) zu gestatten, und durch die, im Gebrauch, die genannten medizinischen Artikel (12) eingeführt werden, **dadurch gekennzeichnet, dass** die genannte erste Detektionsvorrichtung (28), die genannte Steuer- und Verwaltungseinheit (35) und die genannte Druckvorrichtung (39) in dem genannten Halteabteil (16) angeordnet sind, wobei die genannte Trägerstruktur (14) mit einem Schlitz (40) zur Ausgabe des genannten Druckträgers (42) versehen ist, wenn er gedruckt wurde, dadurch, dass die genannte Einführöffnung (22) die einzige Öffnung für einen Zugang zu dem Halteabteil (16) und dem Behälter (24) definiert, dadurch, dass ein Förderrohr (26) mit der genannten Trägerstruktur (14) assoziiert ist, das in Entsprechung zu der Einführöffnung (22) angebracht ist und dafür eingerichtet ist, um die genannten medizinischen Artikel (12) zu dem genannten Behälter (24) zu befördern, dadurch, dass das genannte Förderrohr (26) eine Rohrform aufweist, die durch einen Durchgangshohlraum (27) definiert wird, und dadurch, dass die genannte erste Detektionsvorrichtung (28) einen RFID Leser umfasst, der dafür eingerichtet ist, um die Passage jedes RFID Identifikationselements (13) zu detektieren, das mit jedem der genannten medizinischen Artikel (12) assoziiert ist, wobei die genannte erste Detektionsvorrichtung (28) mit dem genannten Durchgangshohlraum (27) assoziiert ist, der sich über die Gesamtheit seiner Flächenentwicklung erstreckt.

2. Maschine nach Anspruch 1, **dadurch gekennzeichnet, dass** die genannte Steuer- und Verwaltungseinheit (35) mit einer zweiten Detektionsvorrichtung (33) außerhalb der genannten Trägerstruktur (14) verbunden ist und einen RFID Detektor umfasst, um die genannten medizinischen Artikel (12) zu zählen.

3. Maschine nach Anspruch 2, **dadurch gekennzeichnet, dass** die genannte zweite Detektionsvorrichtung (33) vom tragbaren Typ ist.

4. Maschine nach einem der vorhergehenden Ansprüche, **dadurch gekennzeichnet, dass** die genannte Trägerstruktur (14) eine vordere Wand (18) umfasst, die in einer vertikalen oder im Wesentlichen vertikalen Position angeordnet ist, und die genannte Einführöffnung (22) in der genannten vorderen Wand (18) bereitgestellt ist.

5. Maschine nach einem der vorhergehenden Ansprüche, **dadurch gekennzeichnet, dass** sie eine Benutzerschnittstelle (37) umfasst, die in der Trägerstruktur (14) installiert ist, mit der genannten Steuer- und Verwaltungseinheit (35) verbunden ist und dafür eingerichtet ist, um die genannten Daten über den Zustrom medizinischer Artikel (12) anzuzeigen.

6. Maschine nach einem der vorhergehenden Ansprüche, **dadurch gekennzeichnet, dass** die genannte Steuer- und Verwaltungseinheit (35) mit einem internen Speicher (36) versehen ist, um ein Archiv der Zuströme der genannten medizinischen Artikel (12) zu speichern.

7. Maschine nach einem der vorhergehenden Ansprüche, **dadurch gekennzeichnet, dass** die genannte Steuer- und Verwaltungseinheit (35) mit mindestens einer Kommunikationsschnittstelle (38) versehen ist, die dafür eingerichtet ist, um die Daten, die in der Steuer- und Verwaltungseinheit (35) gesammelt werden, an externe Verarbeitungseinheiten zu übertragen.

8. Verfahren zum Zählen medizinischer Artikel (12), welches bereitstellt, medizinische Artikel (12) in einen Behälter (24) einzuführen, der mit einer Trägerstruktur (14) assoziiert ist, um, mittels einer ersten Detektionsvorrichtung (28), mindestens einen Zustrom der genannten medizinischen Artikel (12) zu dem genannten Behälter (24) zu detektieren, um Daten in Bezug auf den genannten Zustrom medizinischer Artikel (12) zu dem genannten Behälter (24) mittels einer Steuer- und Verwaltungseinheit (35) zu erfassen, die mit der genannten ersten Detektionsvorrichtung (28) verbunden ist, und um auf einen Druckträger (42), mittels einer Druckvorrichtung (39), mindestens einige der genannten Daten in Bezug auf mindestens den genannten Zustrom medizinischer Artikel (12) zu drucken, die in den genannten Behälter (24) eingeführt werden, **dadurch gekennzeichnet, dass** die genannte Detektion des genannten Zustroms der genannten medizinischen Artikel (12) mittels eines RFID Lesers erfolgt, der die Passage jedes RFID Identifikationselements (13), das mit jedem der genannten medizinischen Artikel (12) assoziiert ist, durch eine Einführöffnung (22) detektiert, die in der genannten Trägerstruktur (14) bereitgestellt ist, mit einer im Wesentlichen kastenartigen Form, innen hohl, um ein Halteabteil (16) zu definieren, in dem mindestens der genannte Behälter (24) angeordnet ist, dadurch, dass die genannte erste Detektionsvorrichtung (28), die genannte Steuer- und Verwaltungseinheit (35) und die genannte Druckvorrichtung (39) in dem genannten Halteabteil (16) angeordnet sind, wobei der genannte Druckträger (42), wenn er gedruckt wurde, durch einen Schlitz ausgegeben wird, der in der genannten Trägerstruktur (14) bereitgestellt ist, und dadurch, dass die genannte Detektion des Zustroms medizinischer Artikel (12) entlang der gesamten Ausdehnung eines Förderrohrs (26) erfolgt, das mit der Trägerstruktur (14) assoziiert ist, in Entsprechung zu der Einführöffnung (22), und das die genannten medizinischen Artikel (12) zu dem genannten Behälter (24) befördert.

## Revendications

1. Machine pour compter des articles médicaux tels que des éponges chirurgicales utilisées pendant la chirurgie (12), comprenant au moins une structure de support (14) configurée pour supporter au moins un récipient (24) dans lequel, pendant l'utilisation, lesdits articles médicaux (12) sont introduits, un premier dispositif de détection (28) configuré pour détecter au moins un flux desdits articles médicaux (12) vers ledit récipient (24) et une unité de commande et de gestion (35) connectée au moins audit premier dispositif de détection (28) pour acquérir des données au moins relatives audit flux d'articles médicaux (12) vers ledit récipient (24), et comprenant un dispositif d'impression (39) configuré pour imprimer sur un support d'impression (42) au moins certaines desdites données relatives au moins audit flux d'articles médicaux (12) introduits dans ledit récipient (24), ladite structure de support (14) ayant une forme sensiblement en boîte, creuse à l'intérieur, pour définir un compartiment de réception (16) dans lequel au moins ledit récipient (24) est disposé, ladite structure de support (14) étant pourvue d'une ouverture d'introduction (22) configurée pour permettre l'accès audit récipient (24) et à travers lequel, en cours d'utilisation, lesdits articles médicaux (12) sont introduits, **caractérisé en ce que** ledit premier dispositif de détection (28), ladite unité de commande et de gestion (35) et ledit dispositif d'impression (39) sont disposés dans ledit compartiment de réception (16), ladite structure de support (14) étant pourvue d'une fente (40) pour l'émission dudit support d'impression (42) lorsqu'il a été imprimé, **en ce que** ladite ouverture d'introduction (22) définit la seule ouverture pour l'accès au compartiment de réception (16) et au récipient (24), **en ce qu'**une conduite de transport (26) est associé à ladite structure de support (14), fixée en correspondance avec l'ouverture d'introduction (22) et configurée pour transporter lesdits articles médicaux (12) vers ledit récipient (24), **en ce que** ladite conduite de transport (26) a une forme tubulaire définie par une cavité traversante (27) **et en ce que** ledit premier dispositif de détection (28) comprend un lecteur RFID configuré pour détecter le passage de chaque élément d'identification RFID (13) associé à chacun desdits articles médicaux (12), ledit premier dispositif de détection (28) étant associé à ladite cavité traversante (27), s'étendant sur l'ensemble de son développement de surface.

2. Machine selon la revendication 1, **caractérisée en ce que** ladite unité de commande et de gestion (35) est connectée à un second dispositif de détection (33) à l'extérieur de ladite structure de support (14) et comprenant un détecteur RFID pour compter lesdits articles médicaux (12).

3. Machine selon la revendication 2, **caractérisée en ce que** ledit second dispositif de détection (33) est de type portable.

4. Machine selon l'une quelconque des revendications précédentes, **caractérisée en ce que** ladite structure de support (14) comprend une paroi avant (18) située dans une position verticale ou sensiblement verticale, et ladite ouverture d'introduction (22) est pratiquée dans ladite paroi avant (18).

5. Machine selon l'une quelconque des revendications précédentes, **caractérisée en ce qu'**elle comprend une interface utilisateur (37) installée dans la structure de support (14), connectée à ladite unité de commande et de gestion (35) et configurée pour afficher lesdites données sur le flux d'articles médicaux (12).

6. Machine selon l'une quelconque des revendications précédentes, **caractérisée en ce que** ladite unité de commande et de gestion (35) est pourvue d'une mémoire interne (36) pour mémoriser une archive des flux desdits articles médicaux (12).

7. Machine selon l'une quelconque des revendications précédentes, **caractérisée en ce que** ladite unité de commande et de gestion (35) est pourvue d'au moins une interface de communication (38), configurée pour transmettre les données collectées dans l'unité de commande et de gestion (35) à des unités de traitement externes.

8. Procédé pour compter des articles médicaux (12), qui prévoit d'introduire des articles médicaux (12) dans un récipient (24) associé à une structure de support (14), de détecter, au moyen d'un premier dispositif de détection (28), au moins un flux desdits articles médicaux (12) vers ledit récipient (24), d'acquérir des données relatives audit flux d'articles médicaux (12) vers ledit récipient (24) au moyen d'une unité de commande et de gestion (35) connectée audit premier dispositif de détection (28), et d'imprimer sur un support d'impression (42), au moyen d'un dispositif d'impression (39), au moins certaines desdites données relatives au moins audit flux d'articles médicaux (12) introduits dans ledit récipient (24), **caractérisé en ce que** ladite détection dudit flux desdits articles médicaux (12) est effectuée au moyen d'un lecteur RFID qui détecte le passage de chaque élément d'identification RFID (13) associé à chacun desdits articles médicaux (12) à travers une ouverture d'introduction (22) ménagée dans ladite structure de support (14) avec une forme sensiblement en boîte, creuse à l'intérieur, afin de définir un compartiment de réception (16) dans lequel au moins ledit récipient (24) est disposé, **en ce que** ledit premier dispositif de détection (28), ladite unité de commande et de gestion (35) et ledit dispositif d'impression (39) sont disposés dans ledit compartiment de confinement (16), ledit support d'impression (42), lorsqu'il a été imprimé, étant émis à travers une fente prévue dans ladite structure de support (14), **et en ce que** ladite détection du flux d'articles médicaux (12) est effectuée le long de toute l'extension d'une conduite de transport (26) associée à la structure de support (14) en correspondance avec l'ouverture d'introduction (22), et qui transporte lesdits articles médicaux (12) vers ledit récipient (24).
